# EUROPÄISCHE PATENTSCHRIFT

(11) **EP 1 069 889 B1**
(45) Veröffentlichungstag und Bekanntmachung des Hinweises auf die Patenterteilung: **30.06.2004**
(21) Anmeldenummer: 99915750.6
(22) Anmeldetag: 08.04.1999
(51) Int. Cl.: A61K 9/16, A61K 9/72

(54) **PARTIKULÄRER WIRKSTOFFTRÄGER FÜR DIE PULMONALE APPLIKATION**
PARTIKULÄRER WIRKSTOFFTRÄGER FÜR DIE PULMONALE APPLIKATION
EXCIPIENT DE PRINCIPE ACTIF PARTICULAIRE DESTINE A UNE APPLICATION PULMONAIRE

(30) Priorität: 09.04.1998 DE 19816085
(43) Veröffentlichungstag der Anmeldung: 24.01.2001
(73) Patentinhaber: Celanese Ventures GmbH, 65926 Frankfurt am Main (DE)
(72) Erfinder: BENGS, Holger, D-60598 Frankfurt am Main (DE); GRANDE, Jürgen, D-65812 Bad Soden (DE)
(74) Vertreter: Luderschmidt, Schüler & Partner
(86) Internationale Anmeldenummer: PCT/EP1999/002385
(87) Internationale Veröffentlichungsnummer: WO 1999/052506

(56) Entgegenhaltungen:
- WO-A-96/36314
- WO-A-97/35562
- WO-A-99/11695
- GB-A- 937 303

## Beschreibung

Die Erfindung betrifft einen trockenformulierbaren partikulären Wirkstoffträger der aus linearen wasserunlöslichen Polysacchariden hergestellt wird, Verfahren zu seiner Herstellung sowie seiner Verwendung.

In der modernen pharmazeutischen Technologie sind Formulierungen erwünscht, deren Anwendungsform sich auf eine schonende Weise applizieren läßt und gezielt Einfluß auf die Bioverteilung, Bioverfügbarkeit oder Resorption eines Medikaments nehmen.

Vor allem partikuläre Systeme, sogenannte Mikropartikel, die im allgemeinen eine Teilchengröße im Bereich kleiner 100 µm aufweisen, haben sich als Applikationsformen bzw. "Drug Delivery Systeme" hoher Güte erwiesen und dienen zur Bereitstellung von Wirkstoffen, insbesondere Pharmaka, im biologischen Organismus. Dabei haben sich Mikropartikel in einem Größenbereich von 1 bis 100 µm vor allem für die parenterale Anwendung, z. B. die subkutane Injektion bewährt, bei der eine Langzeitabgabe des Wirkstoffs über Wochen erfolgen kann. Wesentlich kleinere Mikropartikel im Bereich kleiner 0,5 µm werden intensiv untersucht, um die Blut-Hirn-Schranke zu überwinden.

Therapeutische Stoffe bedürfen einer Applikationsform, die den physiologischen Gegebenheiten gerecht wird (z. B. therapeutische Proteine u. v. a., die beispielsweise oral appliziert werden, können im Magen denaturieren oder der enzymatischen Verdauung unterliegen, und werden daher zumeist nur als Injektion oder anders invasiv verabreicht).

Insbesondere eine spezielle Form der nichtinvasiven Verabreichung, die pulmonale Applikationsform, bietet im Rahmen einer Inhalationstherapie die Möglichkeit über die Lunge therapeutische Stoffe unter Wahrung der therapeutischen, diagnostischen oder prophylaktischen Eigenschaften zu resorbieren (Patton, Chemtech 1997, 27 (12), 34-38) und ermöglicht die systemische und lokale Behandlung und führt somit zu einer höheren Patienten-Compliance. Hierzu ist es notwendig, partikuläre Systeme in Größe und Qualität bereitzustellen, die in der Lage sind, in Bronchien und Alveolen vorzudringen, wo aufgrund empfindlicher Membranen eine effektive Wirkstoffaufnahme in die Blutbahn des Organismus erfolgt.
Bekannt ist bisher vor allem die bereits am Markt erhältliche pulmonale Applikationsform auf der Basis von treibgasgetriebenen Dosieraerosolen, die nahezu vollständig für die Therapie von asthmatischen Erkrankungen eingesetzt wird. Nachteilig ist jedoch die Verwendung ozonschädigender Treibgase.
Es werden derzeit große Anstrengungen unternommen, um die Applikation von Arzneistoffen über die Lunge auch auf andere Wirkstoffe mit anderen Indikationsgebieten zu übertragen. Im Blickpunkt des Interesses stehen derzeit Pulverinhalationssysteme, sogenannte "Dry Powder Inhaler" auf der Basis einer pharmazeutischen Trockenformulierung. Diese wird in WO 96/32149 beschrieben, hierzu wird als Trägermaterial für den Wirkstoff humanes Serum Albumin einschließlich Zusatzmittel verwendet, Langer et al. beschreiben in Science 276, 1997, 1868 die Verwendung von Mikropartikeln aus Polymilchsäure, die zu 10-20 % mit Insulin beladen werden und vergleichbare Ergebnisse erzielen, wie entsprechend subkutan injiziert (Abstract Controlled Release Society Conference, Sweden 1997). Die Größe der Partikel liegt im Bereich zwischen 8 und 20 µm.

In WO 97/35562 werden wirkstoffbeladene Mikropartikel zur pulmonalen Applikation beschrieben, die aus wäßriger Lösung hergestellt werden und nachteilig nur wasserlösliche Wirkstoffe aufnehmen können.

Eine weitere Druckschrift WO 97/44013 offenbart poröse Partikel aus Polymilch-coglycolsäure, die einen Durchmesser von 5 bis 30 µm aufweisen.

In WO 96/32116 werden Mikropartikel in der Größenordnung von 0,5 bis 50 µm beschrieben, die aus Nukleinsäuren oder viralen Vektoren bestehen und aus einer Suspension mittels hydrophilen oligomeren Polysacchariden unter Trocknung, insbesondere Sprühtrocknung, erhalten werden können.
WO 96/32152 beschreibt Partikel, bestehend aus dem Wirkstoff α-1-Antitrypsin in der Größe von 1-50 µm, wobei auch Verdünnungsmittel zugelassen sind.
Die Druckschrift WO 97/36574 beschreibt hohle Partikel mit glatter Oberfläche, die Größe beträgt 0,5-7,0 µm.

In den Druckschriften WO 97/36574, WO 96/36314, WO 96/00127 und WO 96/32096 werden weitere partikuläre Systeme beschrieben, die aus teilweise synthetischen Polymeren bestehen oder aus dem Wirkstoff als solcher unterschiedlichster Größe.

Im Stand der Technik ist problematisch, daß die Depotwirkung der Partikel relativ begrenzt ist. Des weiteren handelt es sich um wasserlösliche, zumeist aus einer chemischen Synthese hervorgehende Produkte, die deren Biokomptabilität einschränkt (Lösungsmittelreste, Fremdmaterial etc.). Ebenso werden in Größe, Oberfläche und Zusammensetzung unheitliche inhomogene Partikel erhalten, die insbesondere in der pulmonalen Applikation die Bioverfügbarkeit einschränkt, z. B. Verlust durch Ablagerungen außerhalb des Erfolgsortes sowie Beeinträchtigung der Dispergierbarkeit. Des weiteren ergeben sich Probleme im Stand der Technik hinsichtlich der Beladbarkeit des Trägers.

Daher ist es Aufgabe der Erfindung einen trockenformulierbaren Wirkstoffträger besonderer Güte für die auf dem Markt befindlichen Applikatoren zu entwickeln, der diese Nachteile umgeht. Insbesondere sollte dabei die Möglichkeit gegeben sein, eine Partikeltechnik zu etablieren, die in der Lage ist, einen Depoteffekt in der Lunge zu erzielen. Deshalb ist es ebenfalls die Aufgabe der Erfindung dispergierbare partikuläre Wirkstoffträger herzustellen. Ebenfalls eine Aufgabe ist die Bereitstellung eines möglichst einfachen und vorteilhaften Verfahrens zur Herstellung von Trockenformulierungen, die vorzugsweise im Rahmen der pulmonalen Applikation verwendet werden können. Insofern stellt ebenfalls die Optimierung des Wirkstoffträgers in Größe, Oberfläche und Morphologie hinsichtlich der Anwendung zur Inhalation eine Aufgabe dar.
Die Aufgabe wird dadurch gelöst, daß partikuläre Wirkstoffträger zur pulmonalen Applikation bereitgestellt werden, die mindestens ein lineares wasserunlösliches Polysaccharid enthalten und deren mittlere Größe kleiner als 10 µm ist.

Durch die erfindungsgemäßen Maßnahmen werden zusätzliche Vorteile erzielt:
- Leichtere Beladbarkeit des Trägermaterials mit Wirkstoff durch die gute Suspendierbarkeit in verschiedenen Medien (z. B. Dispersionsverfahren, Sprühtrocknung);
- Unbedenklichkeit und hohe Biokomptabilität des verwendeten Trägermaterials durch natürlich vorkommende Strukturen und durch die Verwendung naturidentischer Produkte;
- Die Vermeidung eines Zusatz von oberflächenaktiven Verbindungen bei der Herstellung der Wirkstofformulierung;
- Der für das Flugverhalten sich positiv auswirkende aerodynamische Durchmesser. Dieser wird aufgrund der porösen Oberflächenbeschaffenheit im Vergleich zu einer glatten Kugel erreicht und durch die gleichzeitig mit der Oberflächenrauigkeit einhergehende Tendenz zur Cluster- und / oder Agglomeratbildung unterstützt.

Im Sinne der Erfindung bezeichnet partikulärer Wirkstoffträger Partikel einer mittleren Größe kleiner als 10 µm, insbesondere von 1 bis 10 µm, vorzugsweise 2 bis 6 µm und besonders bevorzugt 1 bis 3 µm, die als essentiellen Bestandteil mindestens ein lineares wasserunlösliches Polysaccharid enthalten.
Lineare wasserunlösliche Polysaccharide im Sinne der vorliegenden Erfindung sind Polysaccharide, vorzugsweise Polyglukane, insbesondere Poly(1,4-alpha-Dglukan), die aus Monosacchariden, Disacchariden, weiteren Oligomeren davon oder Derivaten bestehen.
Diese sind stets in der gleichen Art miteinander verknüpft. Jede so definierte Grundeinheit hat genau zwei Verknüpfungen, jeweils eine zu einem anderen Monomer. Davon sind ausgenommen die beiden Grundeinheiten, die den Anfang und das Ende des Polysaccharids bilden. Diese Grundeinheiten haben nur eine Verknüpfung zu einem weiteren Monomer. Bei drei oder mehr Verknüpfungen (kovalente Bindungen) eines Monomers zu einer anderen Gruppe, bevorzugt einer weiteren Saccharideinheit, spricht man von einer Verzweigung. Von jedem Saccharidbaustein im Polymerrückgrat gehen dann mindestens drei glykosidische Bindungen ab.
Erfindungsgemäß treten Verzweigungen nicht oder nur in so untergeordnetem Maß auf, daß sie bei den vorliegenden sehr kleinen Verzweigungsanteilen im allgemeinen den herkömmlichen analytischen Methoden nicht mehr zugänglich sind. Dies ist zum Beispiel dann der Fall, wenn bezogen auf die Gesamtheit aller vorhandenen Hydroxygruppen auf einhundert Hydroxygruppen, die nicht zum Aufbau des linearen Polysaccharids benötigt werden, maximal fünf Hydroxygruppen durch Verknüpfungen zu anderen Saccharidbausteinen belegt sind.
Dabei ist der Verzweigungsgrad maximal (100 %), wenn an jeder Saccharideinheit die freien Hydroxygruppen (oder andere auftretende funktionelle Gruppen) weitere glykosidische (oder andere) Bindungen zu weiteren Sacchariden aufweisen. Der Verzweigungsgrad ist minimal (0 %), wenn an den Sacchariden außer den Hydroxygruppen, die die Linearität des Polymers bedingen, keine weiteren Hydroxygruppen durch chemische Reaktion verändert sind.
Beispiele für bevorzugte wasserunlösliche lineare Polysaccharide sind lineare Poly-D-glucane, wobei die Art der Verknüpfung unwesentlich ist, solange Linearität im Sinne der Erfindung vorliegt. Beispiele sind Poly(1,4-alpha-D-Glucan) und Poly(1,3-beta-D-Glucan), wobei Poly(1,4-alpha-D-Glucan besonders bevorzugt ist.

Besitzt die Grundeinheit drei oder mehr Verknüpfungen, wird von Verzweigung gesprochen. Dabei ergibt sich aus der Anzahl der Hydroxylgruppen pro 100 Grundeinheit, die nicht am Aufbau des linearen Polymerrückgrats beteiligt sind und die Verzweigungen ausbilden, der sogenannte Verzweigungsgrad.

Erfindungsgemäß weisen die linearen wasserunlöslichen Polysaccharide einen Verzweigungsgrad von weniger als 8 % auf, d.h. sie haben weniger als 8 Verzweigungen auf 100 Grundeinheiten. Vorzugsweise ist der Verzweigungsgrad kleiner 4 % und insbesondere maximal 1,5 %.

Ist das wasserunlösliche lineare Polysaccharid ein Polyglucan, z.B. Poly-(1,4-alpha-D-Glucan), ist der Verzweigungsgrad in 6-Position kleiner 4 %, vorzugsweise maximal 2 % und insbesondere maximal 0,5 % und der Verzweigungsgrad in den anderen nicht an der linearen Verknüpfung beteiligten Positionen, z.B. der 2- bzw. 3-Position im Fall des bevorzugten Poly-(1,4-alpha-D-Glucans), ist vorzugsweise jeweils maximal 2 % und insbesondere maximal 1 %.

Besonders bevorzugt sind Polysaccharide, insbesondere Poly-alpha-D-Glucane, die keine Verzweigungen aufweisen, bzw. deren Verzweigungsgrad so minimal ist, daß er mit herkömmlichen Methoden nicht mehr nachweisbar ist.

Erfindungsgemäß beziehen die Präfixe "alpha", "beta" oder "D" allein auf die Verknüpfungen, die das Polymerrückgrat ausbilden und nicht auf die Verzweigungen.

"Wasserunlöslichkeit" im Sinne der vorliegenden Erfindung bedeutet, daß keine erkennbare Löslichkeit der Verbindung unter Normalbedingungen (Raumtemperatur von 25 °C und ein Luftdruck von 101325 Pascal oder davon maximal 20 % abweichende Werte zugrundeliegt) besteht.

Im Fall der erfindungsgemäß verwendeten Polysaccharide, insbesondere der Polyglukane wie Poly(1,4-alpha-D-glukan), bedeutet dies, daß mindestens 98 % der eingesetzten Menge, bevorzugt eine Menge größer 99,5 %, in Wasser unlöslich ist. Dabei kann der Begriff Unlöslichkeit auch anhand folgender Beobachtung erläutert werden. Erhitzt man 1 g des zu untersuchenden linearen Polysaccharids in 1 I entionisiertem Wasser auf 130 °C unter einem Druck von 1 bar, so bleibt die entstehende Lösung nur kurzzeitig, über wenige Minuten stabil. Beim Erkalten unter Normalbedingungen fällt die Substanz wieder aus. Nach weiterem Erkalten und Abtrennung mit der Zentrifuge unter Einkalkulation von experimentellen Verlusten, lassen sich auf diese Weise mindestens 66 % der eingesetzten Menge zurückgewinnen.
Im Rahmen dieser Erfindung werden bevorzugt lineare, wasserunlösliche Polysaccharide verwendet, welche mit Hilfe von biotechnischen oder gentechnischen Methoden allgemeiner Definition gewonnen werden können. Eine besonders vorteilhafte Ausführungsform für die hier beschriebene Erfindung ist die Herstellung in einem biotechnischen Verfahren, insbesondere in einem biokatalytischen Verfahren, oder in einem fermentativen Verfahren.
Lineare Polysaccharide hergestellt durch Biokatalyse (ebenso: Biotransformation) im Rahmen dieser Erfindung bedeutet, daß das lineare Polysaccharid durch katalytische Reaktion von monomeren Grundbausteinen wie oligomeren Sacchariden, z. B. von Mono- und / oder Disacchariden hergestellt wird, indem ein sogenannter Biokatalysator, üblicherweise ein Enzym, unter geeigneten Bedingungen verwendet wird. Bevorzugt wird insbesondere Poly(1,4-alpha-Dglukan) mittels Polysaccharidsynthasen und / oder Stärkesynthasen und /oder Glykosyltransferasen und / oder alpha-1,4-Glukantransferasen und / oder Glycogensynthasen und / oder Amylosucrasen und / oder Phosphorylasen hergestellt.
Lineare Polysaccharide aus Fermentation sind im Gebrauch der Erfindung lineare Polysaccharide, die durch fermentative Prozesse unter der Verwendung in der Natur vorkommender Organismen, wie Pilze, Algen oder Mikroorganismen oder unter der Verwendung von in der Natur nicht vorkommender Organismen, die durch Modifizierung von natürlichen Organismen, wie Pilze, Algen oder Mikroorganismen, mittels gentechnischer Methoden allgemeiner Definition gewonnen werden können. Darüber hinaus können lineare Polysaccharide zur Herstellung der in der vorliegenden Erfindung beschriebenen Retardtablette aus nicht-linearen Polysacchariden, die Verzweigungen enthalten, gewonnen werden, indem sie mit einem Enzym behandelt werden und unter Spaltung (z. B. mittels Enzyme, wie Amylase, iso-Amylase, Gluconohydrolase, Pullulanase u. a.) und Abtrennung der Verzweigungen lineare Polymere daraus erhalten werden können.

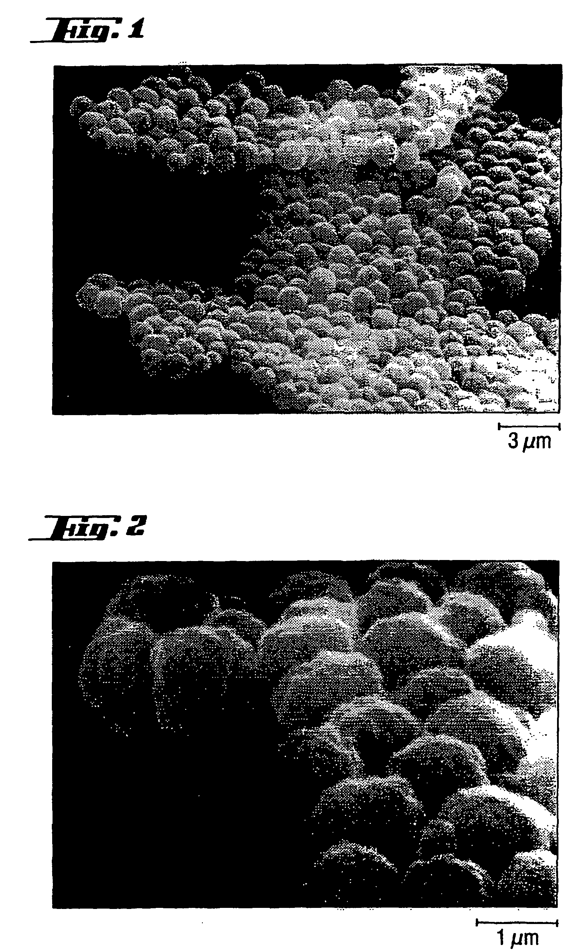

Die Gewinnung und Aufreinigung linearer wasserunlöslicher Polysaccharide mittels bio- und gentechnischen Methoden aus Pflanzen kann nicht ausgeschlossen werden und wird ausdrücklich mit einbezogen.
Die partikuläre Wirkstoffträger können insbesondere durch die Mikropartikeltechnik hergestellt werden, die Gegenstand der Patentanmeldung (DPA, AZ: 197 37 481.6) ist.
Denkbar sind neben der Trockenformulierung Inhalationen auf der Basis von Dispersionen und Suspensionen des Wirkstoffträgers, die hier ausdrücklich mit einbezogen werden. Als besonders geeignet und bevorzugte Ausführungsform ist jedoch die inhalation auf der Basis der Trockenformulierung zu sehen. Hierbei handelt es sich um ein dispergierbares trockenes Pulver, welches mittels Pulverinhalatoren ("Dry Powder Inhaler") pulmonal appliziert werden kann. Erfindungswesentlich ist, daß in der Trockenformulierung Mikropartikel bereitgestellt werden, wie in der Patentanmeldung (DPA, AZ: 197 37 481.6) beschrieben. Hierbei handelt es sich um Monopartikel (Fig. 1 und 2), die einen besonders geeigneten aerodynamischen Durchmesser aufweisen, der sich vorteilhaft auf eine Inhalation auswirkt (z. B. verminderte Abscheidung an Wänden). Besonders vorteilhafte aerodynamischen Durchmesser für die Zwecke der Erfindung zeigen die aus den Monopartikel gebildeten Cluster und / oder Aggregate und / oder Agglomerate. Ebenso wird aufgrund der Mischung dieser Partikel unter Berücksichtigung der anatomischen Gegebenheiten der Lunge eine verbesserte Wirkstoffverteilung und Lungenpenetration (auch: Atemtiefe) erzielt (siehe Beispiele zu Andersen-Impactor). Zur Definition des genannten aerodynamischen Durchmesser bedient sich die Erfindung, der Definition anhand der Schrift WO 97/36574, wobei durch die Multiplikation des geometrischen Durchmessers, der die räumliche Ausdehnung eines kugelförmigen Gebildes (z. B. ein sphärischer Partikel) bestimmt durch den Abstand von Oberfläche zu Oberfläche durch den Mittelpunkt des kugelförmigen Gebildes ermittelt wird, mit der Wurzel der Partikeldichte.
Unter einem Cluster und / oder einem Aggregat und / oder Agglomerat ist eine Anhäufung von Monopartikeln zu verstehen, die durch den Aufbau nichtkovatenter Kräfte entstehen. Besonders vorteilhaft wirkt sich hier beispielsweise eine kappenartige und / oder himbeerartige und / oder sphärische Struktur aus, wie in Bild 3 und Bild 4 dargestellt. Nichtkovalente Kräfte können beispielsweise dipolare Wechselwirkungen, van-der-Waals-Kräfte, Wasserstoffbrückenbindungen oder auch sterische Wechselwirkungen sein, wobei letztere in allgemeiner Definition auch als Schlüsset-Schloss-Prinzip bezeichnet werden können.
Die Molekulargewichte M_{w} der erfindungsgemäß verwendeten linearen Polysaccharide können in einem weiten Bereich von 10³ g/mol bis 10⁷ g/mol variieren, vorzugsweise Molekulargewichte M_{w} im Bereich von 10⁴ g/mol bis 10⁵ g/mol, insbesondere 5 x 10³ g/mol bis 5 x 10⁴ g/mol verwendet. Für das vorzugsweise verwendete lineare Polysaccharid Poly(1,4-alpha-D-glukan) werden entsprechende Bereiche der Molekulargewichte verwendet.
"Biokompatibel" im Sinne dieser Erfindung bedeutet, daß die eingesetzten Polysaccharide einem vollständigen biologischen Abbau unterzogen werden können und weitgehendst keine Anreicherung im Organismus erfolgt.
Unter biologischen Abbau ist dabei jedweder in vivo ablaufende Vorgang angesprochen, der zu einem Abbau oder Zerstörung des Polymers führt Insbesondere fallen ebenfalls hydrolytische oder enzymatische Prozesse in diesen Bereich. Für die Biokompatibilität der Polysaccharide sowie seiner Abbauprodukte (Metabolite) ist nicht zuletzt auch der naturidentische Charakter der eingesetzten Polysaccharide von hoher Bedeutung. Daher sind die in Frage kommenden Polysaccharide für den therapeutischen, diagnostischen oder prophylaktischen Einsatz besonders geeignet.
Des weiteren ergänzt der Begriff "pharmazeutisch akzeptabel" im Sinne dieser Erfindung, daß ein Träger für einen Wirkstoff, ein Hilfsmittel oder auch sogenanntes Excipient, durch ein lebendes Wesen aufgenommen werden kann, ohne daß signifikante Nebenwirkungen für den Organismus entstehen. Im besonderen Fall der pulmonalen Verabreichung bedeutet dies, daß das Hilfsmittel durch die Lunge aufgenommen werden kann und dort durch die körpereigenen Mechanismen abgebaut, aufgelöst, weitertransportiert wird oder aber es zu Ablagerungen kommt, die auch durch Kumulation nicht zu nachteiligen Effekten für das Lebewesen führen. Unter einer "kontrollierten Wirkstoffabgabe" wird verstanden, daß der Wirkstoffe unter Akzeptanz einer den Umständen entsprechenden statistischen Abweichung nach einer bestimmten Zeit und / oder Zeitdauer in einer für den biologischen Organismus vorteilhaften Dosis freigesetzt wird.

Diese Definition beinhaltet auch Extreme. Zum einen die spontane Freigabe aller in der Formulierung vorliegenden Wirkstoffen innerhalb einer auf den Wert Null zusehenden Zeitdauer. Zum anderen die minimal erforderliche Menge/Dosis zur Erzielung eines therapeutischen Effekts über einen langen, gar unendlichen Zeitdauer, mindestens einer Zeitdauer, die notwendig ist, sämtliche in der Formulierung vorliegende Wirkstoffe freizusetzen.
Daher wird für die hier vorliegende Trockenformulierung synonym von einer Depotformulierung oder Formulierung mit verzögerter Freisetzung gesprochen. "Trockenformulierung" und / oder "Puder" im Sinne dieser Erfindung bedeutet eine Komposition, die aus feinen festen Partikeln der jeweiligen pharmazeutischen Zusammensetzung besteht. Diese Partikel sind in ihrer Gesamtheit frei fließend und dispergierbar.
Im besonderen Fall der Inhalationsanwendung bedeutet Trockenformulierung und/oder Puder zusätzlich, daß sie in einem Gerät für die Inhalation für ein trockenes Pulver angewendet werden, so daß ein therapeutischer Effekt erkennbar ist. Man benutzt in diesem Zusammenhang auch den Terminus "anwendbar für den pulmonalen Transport von Wirkstoffen" oder "einatembar", "atembar" oder "respirabel" (engl. "respirable").

In diesem Zusammenhang wird im Rahmen dieser Erfindung unter "trocken" ein Wassergehalt von weniger als 25 % verstanden, wobei ein Wassergehalt von weniger als 15 % bevorzugt ist. insbesondere bevorzugt ist ein Wassergehalt von 5 % bis 10 %.
"Therapeutischer Effekt" im Sinne dieser Erfindung bedeutet, daß eine therapeutisch effektive Menge eines Wirkstoffs an den angestrebten Zielort gelangt, dort seine Wirkung entfaltet, und eine physiologische Reaktion bewirkt Der palliative und / oder kurative Effekt wird einbezogen.

"Dispergierbarkeit" im Sinne dieser Erfindung bedeutet, daß die erhaltene Trockenformulierung durch äußere mechanische Kräfte so zerstäubt oder aufgewirbelt werden kann, daß dieser Zustand für einen bestimmten kurzen Zeitraum so stabil ist (fest in gasförmig oder fest in flüssig), daß ein weitergehender Prozeß eingeleitet werden kann, der einen höheren Nutzen stiftet. Im Rahmen dieser Erfindung dient die pulmonale Anwendung, also die Wirkstoffaufnahme über die Lunge, als herausragendes Beispiel, aber auch andere Wirkstofftransportphänomene sind nicht ausgeschlossen.
Zur pulmonalen Applikation können die allgemein zugänglichen auf dem Markt befindlichen Inhalatoren, insbesondere sogenannte "Dry Powder Inhalatoren" eingesetzt werden sowie bereits in Patenten und Offenlegungen beschriebene Apparaturen verwendet werden (Hersteller: 3M Manufacturing Minnesota Mining, Inc., Inhale Therapeutic Systems, Inc., Dura Pharmaceuticals, Aerogen und Aradigm Corporation, aus: WO 94/16756, WO 96/30068, WO 96/13292, WO 96/33759, WO 96/13290, WO 96/32978, WO 94/08552, WO 96/09085, WO 95/28192).
Die Wirkstoffe, die als pharmazeutische Zusammensetzung mittels des beschriebenen Wirkstoffträgers pulmonal appliziert werden können, unterliegen keinerlei Einschränkungen.
Insbesondere ist die pharmazeutische Zusammensetzung wirksam für Krankheitsbilder, die entweder genetisch bedingt sind oder aber im Lauf des Lebens erworben wurden.
Die Freigabe des Wirkstoffs kann entweder systemisch oder lokal erfolgen. Es kann ein palliativer oder kurativer Effekt angestrebt werden.
Die mit der vorliegenden Erfindung eingesetzten Wirkstoffe können sowohl wasserlöslich als auch wasserunlöslich sein. Die im pharmazeutischen Bereich eingesetzten aktiven Substanzen oder Wirkstoffe können entweder therapeutisch oder diagnostisch, aber auch prophylaktisch aktiv sein.
Die eingesetzten Wirkstoffe können sich auf die verschiedensten indikationen beziehen. Zu nennen sind etwa:
Asthma, zystische Fibrose, allgemeine Lungenkrankheiten, Diabetes (Hypoglykämische Reaktion), Krebs, Mukoviszidose, renale Anämie, Hämophilie, Ovulationsstimulation, Neutropenien, Morbus Gaucher, Hypemephrom, Haarzell-Leukämie, Karzinome, Multiple Sklerose, chronische Granulomatose, Myokardinfarkt, Thrombosen, Hypophysärer Kleinwuchs, Behandlung Heparinassoziierter Thrombozytopenie (HAT) Typ II, Vakzinierungen, Hepatitis B-Prävention, Wachstumsstimulation von zellulären Elementen, Bronchitis, chronische Atemwegserkrankungen, Lungenkrankheiten und Brust Infektionen.
Die partikulären Wirkstoffträger unterliegen keinerlei Einschränkungen hinsichtlich der Stabilität der Wirkstoffe. Bei den verwendeten naturidentischen Polysacchariden handelt es sich um chemisch inerte Substanzen, so daß auch empfindliche Wirkstoffe, wie Peptide, Proteine, appliziert werden können. Hierbei sind insbesondere Peptide und Proteine von Interesse, die aus den zwanzig natürlichen Aminosäuren aufgebaut werden. Der teilweise Ersatz natürlicher Aminosäuren durch nicht natürlich vorkommende Aminosäuren hat jedoch keinen Einfluß auf die Anwendbarkeit der hier beschriebenen Erfindung (z. B. Cetrorelix). Weiterhin ist auch die Applikation von Oligonucleotiden oder viralen Vektoren möglich.
Bei den Proteinen und Peptiden können aus der Natur stammende Verbindungen eingesetzt werden oder aber solche, die sich insbesondere durch bio- oder / und gentechnische Verfahrensschritte herstellen lassen und allgemein unter der Bezeichnung der rekombinaten Wirkstoffe einzugliedern sind. Hier zeichnen sich insbesondere Therapeutika oder Impfstoffe aus, als da sind humane DNAse (Dornase alpha), Erythropoietin alpha (Epoetin alpha), Erythropoietin beta (Epoetin beta), Faktor VII (Eptacog alpha), Faktor VIII, Follitropin alpha, Follitropin beta, G-CSF, glykosyliert (Lenograstim), G-CSF (Filgrastim), GM-CSF (Molgramostim), Glucagon, Glucocerebrosidase (Alglucerase), IL-2 (Aldesleukin), Interferon alpha-2a, Interferon alpha-2b, Interferon beta-1b, Interferon gamma-1b, Insulin, (Humaninsulin, Lispro), t-PA (Alteplase), r-PA (Reteplase), humane Wachstumshormon (HGH, Somatropin), Hirudin, Hepatitis B-antigen , Hepatitis A/B Kombinationsimpfstoff, MPIF-1 (Myeloid Progenitor Inhibitor Factor-1), KGF-2 (Keratinozyten-Wachstumsfaktor).

Insgesamt können auch Wirkstoffe aus der folgenden Gruppe und Klassen dieser Wirkstoffe vorteilhaft mit der hier vorgestellten Technologie vor allem in der pulmonalen Applikation zur Anwendung gelangen:
Calcitonin, Felbamat, AZT, DDI, GCSF, Lamotrigin, Gonadotropin Releasing Faktor (Hormon) (GNRH, GHRH und GHRH-Analoga), Luteinisierendes Hormon Releasing Hormon (LHRH) und LHRH Analoga (sowohl Antagonisten als auch Agonisten, z. B. Leuprolid Acetat, Leuprolid, Lupron or Lupron Depot, Buserelin, Ramorelix, Cetrorelix), TRH (Thyreotropin Releasing Hormon), Adenosine Deaminase, Argatroban, α1-Antitrypsin, Albuterol, Amilorid, Terbutalin, Isoproterenol, Metaprotaranol, Pirbuterol, Fluticason Propionat, Budesonied, Beclomethasone Dipropionate, Cromoglycinsäure, Dinatriumcromoglycinat, Nedocromil, Sultanol, Beclomethason, Bambuterol, Mometason, Triacetonid, Isoproterenol, Cromolyn, Salmeterol, Salmeterol Xinotat, Formotorol, Triamcinolon Acetonid, Flunisolid, Fluticason, Salbutamol, Budesonid, Fenoterol, Ipratropiumbromid, Tachykinin, Tradykinin, Furosemid, Dinatriumcromoglycat, Nafarelin, Cromolyn Natrium, Albuterolsulfat, Metaproterenolsulfat, Somatostatin, Oxytocin, Desmopressin, ACTH Analoga, secretin glucagon, Kodein, Morphin, Diltiazem, Cromoglycat, Ketotifen, Cephalosporin, Pentamidin, Fluticason, Tipredan, Noscapine, Isoetharin, Amilorid, Ipratropium, Oxitropium, Cortison, Prednisolon, Aminophylin, Theophylin, Methapyrilen.
Analgetika, Anginal Präparationen, Antiallergika, Antihistamine, Anti-inflammatories, Bronchodilatoren, Bronchospasmolytika, Diuretika, Anticholinergetika, Anti-Adhäsions Moleküle, Zytokin Modulatoren, biologisch aktive Endonucleasen, rekombinante Human-DNasen, Neurotransmitter, Leukotrin Inhibitoren, vasoaktive Intestinal Peptide, Endothelin Antagonisten, Analeptika, Analgetika, Lokalanästhetika, Narkosemittel, Antiepileptika, Antikonvulsiva, Antiparkinsonmittel, Antiemetika, das Hormonsystem regulierende oder stimulierende Verbindungen, das Herz-Kreislauf-System regulierende oder stimulierende Verbindungen, den Respirationstrakt System regulierende oder stimulierende Verbindungen, Vitamine, Spurenelemente, Antioxidantien, Zytostatika, Antimetabolite, Antiinfektiva,
Immunmodulatoren, Immunsuppressiva, Antibiotika, Proteine, Peptide, Hormone, Wachstumshormone, Wachstumsfaktoren, Xanthine, Vakzine, Steroide, b₂-Mimetika.
Es können sowohl entweder die freien Säuren oder Basen, aber auch pharmazeutisch aktive und akzeptable Salze dieser Verbindungen und Substanzklassen, sowie deren Hydrolysate oder Bruchstücke oder Metabolite davon verwendet werden.
Ebenso können für die vorliegende Formulierung übliche Hilfs- und Zusatzstoffe verwendet werden.
Die nachfolgenden Beispiele dienen zur näheren Erläuterungen der Erfindung, ohne dieselbe auf in den Beispielen beschriebenen Produkte und Ausführungsformen einzuschränken.

### Beispiele

Die folgende Beispiele beziehen sich insbesondere auf die Herstellung der Mikropatikel, wie in der Patentanmeldung (DPA, AZ: 197 37 481.6) beschrieben, auf diese wird ausdrücklich bezug genommen. Des weiteren wird eine besonders vorteilhafte Methode zur Herstellung von Poly(1,4-alpha-D-glukan) in WO 95/31553 beschrieben.
Zudem werden Beispiele zur Verdeutlichung der pulmonalen Applikation insbesondere mit Hilfe der Trockenformulierung im Rahmen der Pulverinhalation angeführt.

### Beispiel 1

### In-vitro-Produktion von Poly(1,4-α-D-glukan) in einem biokatalytischen Prozeß mit Hilfe des Enzyms Amylosucrase

In einem sterilisierten (Dampfsterilisation) 15 I Gefäß werden 10 I einer 20 %igen Saccharose Lösung gegeben. Der mittels einer Fermentation erhaltene Amylosucrase enthaltende Enzymextrakt wird in einer Portion zu der Saccharoselösung gegeben. Die Enzymaktivität beträgt 16 units (1 unit entspricht der Umsetzung von 1 µmol Saccharose pro Minute pro mg Enzym). Die Apparatur wird mit einem ebenfalls sterilisierten KPG-Rührer versehen. Das Gefäß wird verschlossen und bei 40 °C aufbewahrt und gerührt. Nach einiger Zeit bildet sich ein weißer Niederschlag. Die Reaktion wird nach einer Zeitdauer von 180 Stunden beendet. Der Niederschlag wird abfiltriert und zur Abtrennung niedermolekularer Zucker mehrfach gewaschen. Der im Filter verbleibende Rückstand wird bei Temperaturen zwischen 30 und 40 °C im Trockenschrank unter Anlegung eines Vakuums mit Hilfe einer Membranpumpe (Firma Vacuubrand GmbH & Co, CVC 2) getrocknet. Die Masse beträgt 685 g (Ausbeute 69 %).

### Beispiel 2

### Charakterisierung des mit Amylosucrase synthetisierten Poly(1,4-α-D-glukan) aus Beispiel 1 mittels Gelpermeationschromatographie

Es werden 2 mg des Poly(1,4-α-D-glukan) aus Beispiel 1 bei Raumtemperatur in Dimethylsulfoxid (DMSO, p. a. von Riedel-de-Haen) gelöst und filtriert (2 mm Filter). Ein Teil der Lösung wird in eine Gelpermeationschromatographie Säule injeziert. Als Elutionsmittel wird DMSO verwendet. Die Signalintensität wird mittels eines RI-Detektors gemessen und gegen Pullulanstandards (Firma Polymer Standard Systems) ausgewertet. Die Flußrate beträgt 1.0 ml pro Minute.
Ein Meßergebnis ist im folgenden angegeben: Zahlenmittel des Molekulargewichts (Mₙ) von 3.200 g/mol und ein Gewichtsmittel des Molekulargewichts (M_{w}) von 9.300 g/mol. Dies entspricht einer Dispersität von 2,8

### Beispiel 3

### Herstellung von Mikropartikeln aus Poly(1,4-α-D-glukan)

400 g Poly(1,4-α-D-glukan) werden in 2 I Dimethylsulfoxid (DMSO, p. a. von Riedel-de-Haen) bei 40 °C innerhalb von gelöst. Dann wird eine Stunde bei Raumtemperatur gerührt. Die Lösung wird in 20 l bidestilliertem Wasser unter Rühren durch einen Tropftrichter über einen Zeitraum von 2 h hinzugegeben. Der Ansatz wird für 40 h bei 6°C gelagert. Es bildet sich eine feine Suspension aus. Der Partikel werden abgetrennt, indem zunächst der Überstand abdekantiert wird. Der Bodensatz wird aufgeschlämmt und in kleinen Portionen zentrifugiert (Ultrazentrifuge RC5C: je 5 Minuten bei 5000 Umdrehungen pro Minute). Der feste Rückstand wird insgesamt drei Mal mit bidestilliertem Wasser aufgeschlämmt und erneut zentrifugiert. Die Feststoffe werden gesammelt und die Suspension von ca. 1000 ml gefriergetrocknet (Christ Delta 1-24 KD). Es werden 283 g weißer Feststoff isoliert (Beispiel 3a: Ausbeute 71 %). Die gesammelten Überstände werden bei einer Temperatur von 18 °C über Nacht verwahrt. Die Aufarbeitung erfolgt wie beschrieben. Es werden weitere 55 g des weißen Feststoffs isoliert (Beispiel 3b: Ausbeute 14 %). Die Gesamtausbeute beträgt 85 %.

### Beispiel 4

### Entschwefelung der Mikropartikel aus Beispiel 3

Zur Abtrennung in den Partikeln verbliebenen Dimethylsulfoxids wird wie folgt vorgegangen. 100 g der Amylosepartikel aus Beispiel 9 werden in 1000 ml entionisiertem Wasser gegeben. Der Ansatz wird für 24 h unter leichtem Schwenken sich selbst überlassen. Die Abtrennung der Partikel erfolgt wie in Beispiel 9 beschrieben (Ultrazentrifuge RC5C: je 15 Minuten, 3000 U / min. Nach der Gefriertrocknung ergibt sich eine Auswaage von 98,3 g (98 % Ausbeute).
Die Schwefelbestimmung durch Elementaranalyse ergibt folgende Werte (Prüfmethode Verbrennung und IR-Detektion):

| | |
|---|---|
| Schwefelgehalt der Partikel aus Beispiel 2 | 6 % +/- 0,1 % |
| Schwefelgehalt der Partikel aus Beispiel 3 | < 0,01 % |

### Beispiel 5

### Untersuchungen der Mikropartikel aus dem Beispiel 3 mittels Elektronenmikroskopie

Zur Charakterisierung der Partikel werden Rasterelektronenmikroskopaufnahmen (REM) (Camscan S-4) durchgeführt. Die Bilder (Fig. 1 und 2) zeigen Aufnahmen der Partikel, die verdeutlichen, daß es sich um sphärische, sehr einheitliche Partikel hinsichtlich der Form, Größe und Oberflächenrauigkeit handelt.

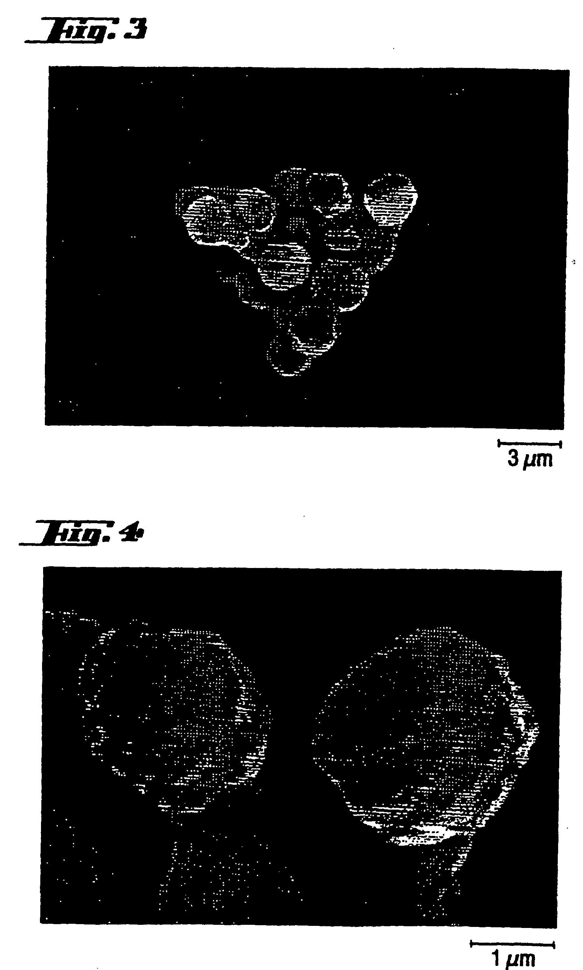

Die Bilder (Fig. 3 und 4) zeigen Aufnahmen der bebildeten Cluster und / oder Agglomerate.

### Beispiel 6

### Untersuchungen der Größenverteilungen der Partikel aus Beispiel 3

Zur Charakterisierung der Größenverteilungen der Partikel aus den Beispielen 1 und 9 wurden Untersuchungen mit einem Mastersizer durchgeführt (Fa. Malvem Instruments). Die Untersuchung erfolgte im Fraunhofer Modus (Auswertung: multimodal, Anzahl) mit einer Dichte von 1,080 g/cm³ und Volumenkonzentration im Bereich von 0,012 % bis 0,014 %.

**Tabelle 1:**

| Charakterisierung der Partikeldurchmesser der Mikropartikel aus Beispiel 3. | | | | | | |
|---|---|---|---|---|---|---|
| Beispiel | Durchmesser | | | Partikelverteilung | | |
| No. | Dn*¹ (mm) | dw*² (mm) | dw/dn*³ | d (10 %)*⁴ (mm) | d (50 %)*⁵ (mm) | d (90 %)*⁶ (mm) |
| 3a | 1,664 | 4,184 | 2,541 | 0,873 | 1,504 | 2,624 |
| 3b | 0,945 | 2,345 | 2,481 | 0,587 | 0,871 | 1,399 |

| | | | | | | |
|---|---|---|---|---|---|---|
| *¹ dn: Zahlenmittelwert des Durchmessers | | | | | | |
| *² dw: Gewichtsmittelwert des Durchmessers | | | | | | |
| *³ dw / dn: Dispersität der Partikeldurchmesser | | | | | | |
| *⁴ d(10 %): 10 % aller Partikel haben einen kleineren Durchmesser als der angegebene Wert | | | | | | |
| *⁵d(50 %): 50 % aller Partikel haben einen kleineren Durchmesser als der angegebene Wert | | | | | | |
| *⁶ d(90 %): 90 % aller Partikel haben einen kleineren Durchmesser als der angegebene Wert | | | | | | |

### Beispiel 7

### Bestimmung der Flugweite von Mikropartikeln aus Poly(1,4-α-D-glukan)

Andersen Impactor (Andersen Sampiers Inc., 4215 Wendell Drive, Atlanta, GA, USA und vgl. Pharmacopoeia Europaeum, Stuttgart, Deutscher Apothekerverlag 1997, kap. 2.9.18; dort: Gerät D) besteht aus einer Kaskade von voneinander abgetrennten Platten, die sukzessiv mit kleineren Löchern (Poren) ausgestattet sind. Abhängig von der aerodynamischen Partikelgröße findet auf den einzelnen Platten eine Ablagerung statt.

**Tabelle 2:**

| Charakteristik des Andersen Impactors | | | |
|---|---|---|---|
| Filterstufe | Lochdurchmesser [inch] | Lochdurchmesser [µm]*¹ | Zahl der Löcher |
| 0 | 0,1004 | 2550 | 96 |
| 1 | 0,0743 | 1887 | 96 |
| 2 | 0,0360 | 914 | 400 |
| 3 | 0,0280 | 711 | 400 |
| 4 | 0,0210 | 533 | 400 |
| 5 | 0,0135 | 342 | 400 |
| 6 | 0,0100 | 254 | 400 |
| 7 | 0,0100 | 254 | 201 |

| | | | |
|---|---|---|---|
| *¹Die Angaben in "mm" wurden durch Umrechnung der Herstellerangaben in "inch" erhalten. | | | |

Die Andersen-Impactor Experimente verlaufen wie folgt: 100 mg der Substanz (hier: drei verschiedene Proben von Mikropartikeln und Agglomeraten (Cluster) aus Poly(1,4-α-D-glukan); hergestellt nach Bsp. 3 und 4), werden in den Ansatzstutzen des Impactors gegeben und mit der zur Ausrüstung gehörenden Pumpe in die Filterkaskade hineingesaugt (Pumpenleistung: 35 l/min.). Nach einer Minute wird das Gerät gestoppt und die Masse der einzelnen Filter (oder Prallplatten) bestimmt. Die Massen an Mikropartikeln sind in der folgenden Abbildung aufgetragen. Dabei wird vor allem das gute Flugverhalten bei minimaler Pumpenleistung deutlich, welches sich in der hohen Masse auf höheren Prallplatten (Filtern) widerspiegelt. Der am Startpunkt verbleibende Rest kann minimiert werden, indem die Partikel durch mechanische Vibration am Ansatzstutzen des Impactors in der Schwebe gehalten werden (vgl. Probe 2).

Das Verhalten verschiedener Proben von Poly(1,4-α-D-glukan) im Andersen Impactor ist in Fig. 5 gezeigt

### Beispiel 8

### Bestimmung der Flugweite von Pudern aus weiteren Polysacchariden, herkömmlichen Inhalern und Mikropartikeln aus synthetischen Polymeren (Vergleichsbeispiele)

Die Vergleichsbeispiele mit anderen, teils wasserlöslichen Polysacchariden, Material aus herkömmlichen Inhalern und Mikropartikeln aus synthetischen, aber bioabbaubaren Polymeren (Vergleichsbeispiele) wurden analog zu Beispiel 8 durchgeführt. Bei den Polysacchariden handelt es sich um eine Kartoffelstärke des Typs Toffena (Fa. Südstärke) und eine Reisstärke des Typs Remygel (Fa. REMY) mit Partikelgrößen bis zu 100 mm und um 4 mm. Bei den Inhalern handelt es sich um den Aerolizer (inhaler 1, Fa. Ciba Geigy) und den Atemur Diskus (Inhaler 2, Fa. cascan). Die Mikropartikel aus Polylactid-co-glycolid (PLGA) wurden mittels Sprühtrocknung (Material PLGA 65:35-d, I der Fa. Medisorb) hergestellt und haben ca. Durchmesser zwischen 2 und 15 mm.

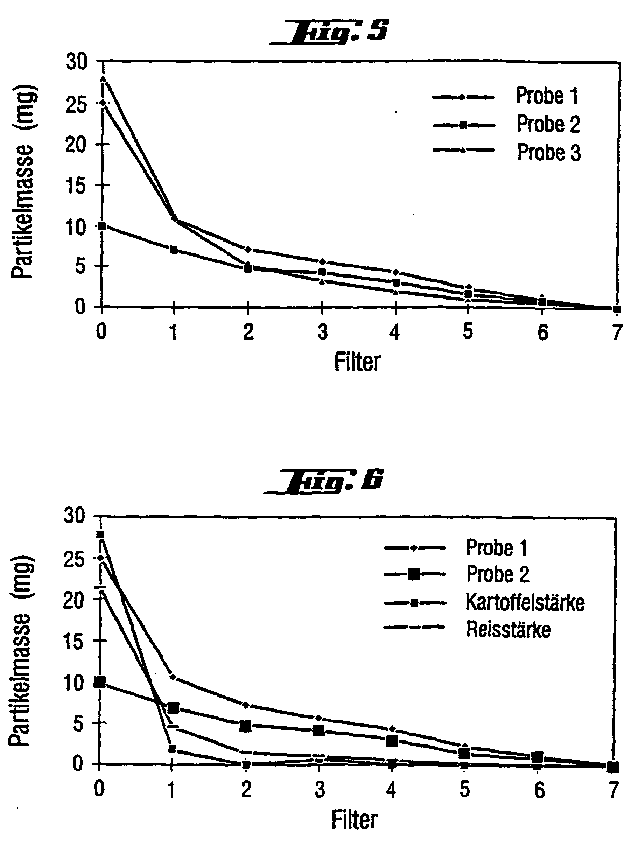

Das Verhalten anderer, teils wasserlöslicher Polysaccharide im Andersen Impactor (Vergleichsbeispiele) ist in Fig. 6 gezeigt.

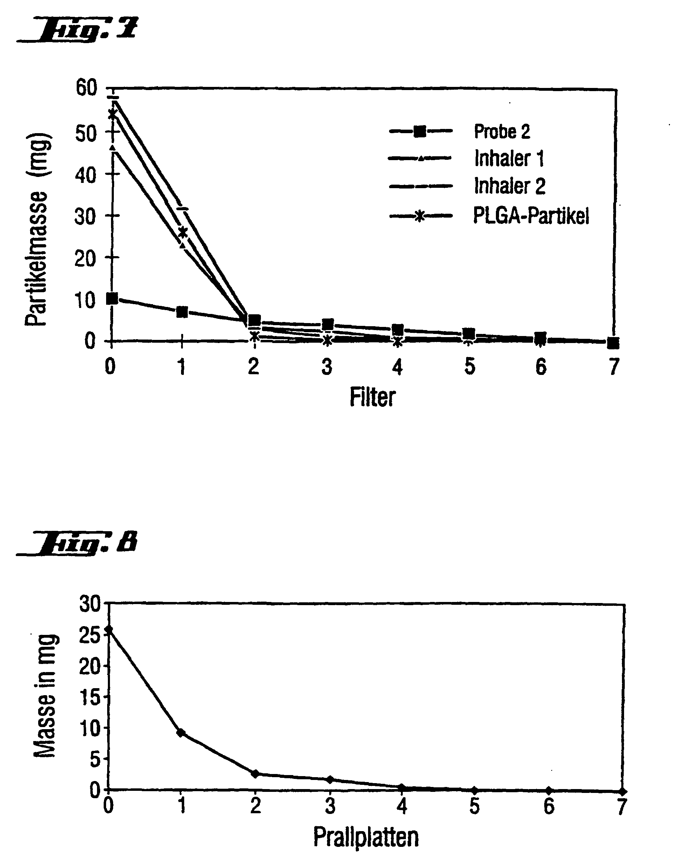

Das Verhalten von Material herkömmlicher Inhaler und Mikropartikeln aus synthetischen, bioabbaubaren Polymeren im Andersen Impactor (Vergleichsbeispiele) ist in Fig. 7 gezeigt

### Beispiel 9

### Bestimmung der Flugweite von Mikropartikeln verschiedener Geometrien aus Poly(1,4-α-D-glukan) und Agglomeraten daraus

Es wird das Flug- oder Abscheideverhalten von Mikropartikeln verschiedener Größe und Oberflächenrauigkeit in einem gekrümmten Rohr untersucht. Die Versuche bilden die Grundlage für eine Abschätzung einer optimalen Morphologie von Partikeln und Agglomeraten für die Applikation in einem Dry Powder Inhaler. Folgende Annahmen liegen dem Modell zugrunde: luftdurchströmter Kanal d = 20 mm; Luftdichte r_{fl} = 1,2 kg/m³; Luftviskosität h = 1,81 x 10⁻⁵ Pa s; Strömungsgeschwindigkeit v_{fl} = 5 m/s; Krümmungsradius R = 20 (200) mm; Krümmungswinkel a = 45°; Partikeldurchmesser x = 1 - 5 mm; Feststoffdichte rₛ = 1550 kg/m³; Formfaktor Y = 1 und abweichend.
Zunächst wird das Verhalten kugeliger und nichtkugeliger Partikeln zueinander vergleichen. Für nichtkugelige Partikeln wird eine Struktur zur Beschreibung der Geometrie vorgegeben. Gesucht sind die Sinkgeschwindigkeit im Schwerefeld in Luft sowie die radiale Auslenkung eines solchen Partikels bei Durchströmung eines Rohrbogens. Anhand der berechneten Auslenkung kann abgeschätzt werden, wie viele Teilchen bei der Durchströmung des Rohrbogens die Wand berühren und haften bleiben, also abgeschieden werden.

Die Abweichung der Partikelform von der Kugel läßt sich mit Hilfe des Formfaktors Ψ beschreiben. Er ist definiert als das Verhältnis der Oberfläche einer Kugel mit gleichem Volumen zur tatsächlichen Oberfläche des Partikels. Für Kugeln ist Ψ = 1, für nichtkugelige Partikeln gilt Ψ < 1. Je kleiner der Formfaktor wird, um so eher folgt ein Teilchen der Luftströmung und um so weniger wird es durch Wandhaftung in Rohrkrümmungen abgeschieden. Der aerodynamische Durchmesser verhält sich reziprok zum Formfaktor. Ein Wert von Ψ = 0,8 ist zum Beispiel mit einer Absenkung der Feststoffdichte von 1550 kg/m³ auf 1400 kg/m³ vergleichbar.
In einer ersten Betrachtung wurde eine glatte Kugel mit einer Kugel mit rauher Oberfläche verglichen. Die Rauheit wurde durch kleinere Kugelabschnitte (Kugelkappen), die auf eine Basiskugel aufgesetzt wurden, angenähert. Die Abschnitte waren einerseits nach außen (Himbeerstruktur) andererseits nach innen (Kraterlandschaft) gerichtet. Für nach außen gerichtete Abschnitte ergab sich der Formfaktor zu Ψ = 0,958, für nach innen gerichtete zu Ψ = 0,946. Weiterhin wurden Agglomerate aus Partikeln betrachtet. Die betrachteten Agglomerate (Körper) bilden idealerweise folgende Anordnungen in der Anordnung einer dichtesten Kugelpackung (für Tetraeder vgl. auch Abbildung 2). Die Körper werden aus Agglomeraten einzelner Kugeln modelliert und besitzen folgende Struktur: a) ein aus Kugeln aufgebauter Tetraeder mit 4 Schichten. Die Kanten werden aus jeweils 4 Kugeln gebildet. Insgesamt besteht der Tetraeder aus 20 Einzelkugeln; b) Körper aus drei Kugelschichten mit 3, 7 und nochmals 3 Einzelkugeln (kleiner Doppelkegel); c) Körper aus 5 Schichten mit einer Kugelanzahl von 1, 3, 7, 3 und 1 je Schicht (großer Doppelkegel).
Die Tabelle faßt die Ergebnisse der Berechnungen zusammen. Als Parameter wird für die Vergleichbarkeit nur die Abweichung von der Kugelform bei einem vorgegebenen vofumenäquivalenten Partikeldurchmesser von 5 µm betrachtet. Es ist zu beachten, daß der Durchmesser der Einzelkugeln für die o. a. Geometrien entsprechend geringer ist. Die Sinkgeschwindigkeit nimmt mit zunehmender Abweichung von der Kugelform ab. Demzufolge verringert sich auch der Abscheidegrad bei der Durchströmung eines Rohrbogens. Im Vergleich zur Kugel mit dem Durchmesser von 5 µm reduziert sich der Abscheidegrad für die aus Agglomeraten aufgebauten Partikelformen auf 66 %. Die Ergebnisse dieser Modellversuche belegen die Vorteilhaftigkeit der vorliegenden Erfindung hinsichtlich der Oberflächenrauigkeit, sowie der Agglomerate, die im Sinne einer dichtesten Kugelpackung organisiert sind. Insbesondere zeigt sich, daß mit fallendem Formfaktor Ψ, d. h. gegen Null, der aerodynamische Durchmesser sich verbessert.
Je besser der aerodynamische Durchmesser desto tiefer die Lungenpenetration (Atemtiefe).

**Tabelle 2:**

| Ergebnisse der Berechnung für die oben beschriebenen Partikelformen | | | | | |
|---|---|---|---|---|---|
| | Formfaktor Ψ | Agglomeratdichte [kg/m³] | Sinkgeschwindigkeit im Schwerefeld [m/s] | radiale Auslenkung [mm] | Abscheidegrad [%] |
| Kugel | 1 | 1550 | 0,001165 | 0,466 | 2.33 |
| Kugel (Kappen innen) | 0,946 | 1550 | 0,001134 | 0,453 | 2,26 |
| Kugel (Kappen außen) | 0,958 | 1550 | 0,001141 | 0,456 | 2,28 |
| Agglomerat Tetraeder | 0,49 | 1430 | 0,000753 | 0,301 | 1,50 |
| Agglomerat Doppelkegel 3 Ebenen | 0,56 | 1390 | 0,000782 | 0,313 | 1,56 |
| Agglomerat Doppelkegel 5 Ebenen | 0,52 | 1405 | 0,000762 | 0,305 | 1,52 |

### Beispiel 10

### Beladung der Partikel mit Wirkstoff durch ein Suspensionsverfahren

Die Mikropartikel, bzw. Agglomerate, aus Poly(1,4-α-D-glukan) (Herstellung in Beispiel 3 und 4) werden durch einen Suspensionsprozeß mit Wirkstoff beladen. 250 mg Buserelin* werden in 10 ml destilliertem Wasser gelöst. Es werden 100 mg Partikel hinzugegeben. Die Suspension wird 3 h gerührt. Die Suspension wird zentrifugiert. Das Zentrifugat wird mit Wasser gewaschen. Der partikuläre Feststoff wird per Zentrifuge abgetrennt (3000 U/min) und das Zentrifugat gefriergetrocknet. Durch Auflösung einer exakten Menge der Partikel in einem Wasser-Dimethylsulfoxid und der spektroskopischen Vermessung im UV-Vis-Spektrometer kann über eine Eichkurve die Beladung mit Buserelin zu 3,28 % bezogen auf die Gesamtmasse der Partikel berechnet werden. Durch die Modifizierung des Lösungsmittels für den Wirkstoff, z. B. Alkohole, kann die Löslichkeit und damit die Beladung der Partikel mit Wirkstoff beeinflußt werden.

### (*5-Oxo-L-prolyl-L-histidyl-L-tryptophyl-L-seryl-L-tyrosyl-O-tert-butyl-D-seryl-Lleucyl-L-arginyl-N-ethyl-L-prolinamid)

### Beispiel 11

### Beladung der Partikel mit Wirkstoff mittels Sprühtrocknung

Die Mikropartikel werden in destilliertem Wasser, bzw. einer Mischung aus Wasser und einer leicht flüchtigen Komponente wie Aceton oder Ethanol, suspendiert. Hierzu werden 10 g des Feststoffs in 1 000 ml des Lösungsmittels suspendiert. In dem Lösungsmittel wurden zuvor 0,5 g Theophyllin aufgelöst. Der Sprühtrockner (Mini Sprühtrockner 191 der Fa. Büchi) wird wie folgt betrieben:
Zerstäubungsluftstrom 700 Liter pro Stunde, Eingangstemperatur 200 °C, eingeschaltete Düsenkühlung, Düsendurchmesser 0,5 mm, Aspirator 70 %, Pumpe 10 %. Die spektroskopische Oberprüfung der Beladung (Beschreibung siehe Beispiel oben) ergibt einen Beladungsgrad von 4,8 %. Dieser Wert stimmt mit dem theoretisch erreichbaren Wert von 5,0 % im Rahmen der Fehlergrenzen überein.

### Beispiel 12

### Bestimmung der Löslichkeit von Polysacchariden

Es werden 100 mg Poly(1,4-α-D-glukan) in 5 ml bidestilliertem Wasser gegeben.
Das Reaktionsgefäß wird unter Rühren (Magnetrührer) langsam aufgehetzt. Es wird in einem Stufenprogramm mit Abständen von zwanzig Grad erhitzt und mit dem Auge beobachtet. Bei Temperaturen von 40 °C, 60 °C, 80 °C und 100 °C sind keine Veränderungen zu beobachten. Gemäß dieser Beobachtungen ist die Verbindung die Eigenschaft "wasserunlöslich" zuzuordnen.

### Beispiel 13

### Bestimmung der Löslichkeit von Polysacchariden und Klassifizierung nach Deutschem Arzneibuch (DAS)

564 mg Poly(1,4-α-D-glukan) werden in ca. 0,5 L bidestilliertem Wasser bei 1,3 bar und 130 °C für 1,5 Stunden in einem Autoklaven erhitzt (Apparat Certoclav). Von dem Reaktionsgefäß ist zuvor das Gewicht gemessen worden. Danach wird die Apparatur entspannt und bei Raumtemperatur abgekühlt. Der Inhalt wird gewogen. Er entspricht 501,74 g. Nach weiteren 24 Stunden wird zentrifugiert und dekantiert. Der feste Rückstand wird getrocknet und ausgewogen: 468 mg. Daraus errechnet sich ein gelöster Anteil von 96 mg. Bezogen auf das eingesetzte Lösungsmittel errechnet sich daraus, daß für 1 mg Poly(1,4-α-D-glukan) 5226 mg Wasser notwendig sind. Gemäß der Klassifizierung nach Deutschem Arzneibuch ergibt sich daraus die Einteilung, daß diese Substanz "sehr schwer löslich" ist, da zwischen 1.000 und 10.000 Teilen Lösungsmittel notwendig sind, um 1 Teil der Substanz in Lösung zu bringen. Dies ist von den 7 Klassen zur Einteilung der Löslichkeit (von "sehr leicht löslich" (Klasse 1) bis "praktisch unlöslich"(Klasse 7) die Klasse Nummer 6.

### Beispiel 14

### Bestimmung der Löslichkeit von Polysacchariden und Klassifizierung nach Deutschem Arzneibuch (DAB)

Der Versuch wird wie in Beispiel 13 durchgeführt. Der einzige Unterschied bildet ein Kühlprozeß, der nach der Autoklavbehandlung und dem Abkühlen auf Raumtemperatur nachgeschaltet wird. Das Substanzgemisch wird für 3 Stunden bei 5 °C aufbewahrt.

Es werden 526 mg Poly(1,4-α-D-glukan) auf ca. 480 mL bidestilliertem Wasser eingewogen. Nach der thermischen Behandlung ergibt sich eine Auswaage von 468,09 g. Das getrocknete Sediment beträgt 488 mg. Demnach sind 38 mg des Poly(1,4-α-D-glukans) in Lösung gegangen. Dies entspricht einem Verhältnis von 1 mg Substanz zu 12.318 Teilen Lösungsmittel. Demnach ist die Substanz nach dieser Behandlungsmethode in Klasse Nummer 7 nach DAB einzustufen und danach als praktisch unlöslich zu klassifizieren, weil mehr als 10.000 Teile Lösungsmittel für ein Teil Substanz benötigt werden.

### Beispiel 15

### Herstellung von Mikropartikeln aus einer autoklavierten Poly(1-4-alpha-D-glukan)-Suspension

3,5g Poly(1-4-alpha-D-glukan)-Pulver werden dreimal mit 60°C heißem Wasser gewaschen. Anschließend wird in 200 ml entionisiertem Wasser suspendiert. Die Suspension wird in einem Laborautoklaven (Pressure Vessels; Typ 452 HCT 316; Fa. Parr Instruments Deutschland GmbH) angesetzt. Die Kammer wird mit Stickstoff gespült, so daß ein Druck von 1,5 bar erreicht wird.
Unter Rühren wird der Autoklav auf 130°C erwärmt. Die Autoklavierzeit bei dieser Temperatur beträgt 30 min. Es wird ein Druck von ca. 5 bar erzeugt.
Nach dem Abkühlen des Autoklavs wird die Suspension entnommen und sofort im Sprühtrockner versprüht. Dabei wird die Suspension mit Hilfe eines Magnetrührers kontinuierlich gerührt.
Der Sprühtrockner (Mini Sprühtrockner 191, Fa. Büchi, Schweiz) wird wie folgt betrieben: Zerstäubungsstickstoffstrom 700 Liter pro Stunde, Eingangstemperatur 220°C, eingeschaltete Düsenkühlung, Düsendurchmesser 0,5mm, Aspirator 70%, Pumpe 70%.
Das Sprühtrocknungsgut wird dem Auffanggefäß des Zyklons entnommen und trocken gelagert. Die Größe der Mikropartikel liegt zu über 90 % in einem Bereich kleiner 10 µm (Auswertung von Elektronenmikroskop-Aufnahmen).

### Beispiel 16

### Herstellung von Mikropartikeln aus Poly(1,4-alpha-D-glukan)

Es wird die Möglichkeit untersucht, direkt aus der Biotransformation anfallendes Polyglukan für die Herstellung lungengängiger Partikel herzustellen. Hierzu wird eine 20 %ige Suspension hergestellt, indem 500 g Poly(1,4-alpha-D-glukan) aus Beispiel 1 in 2,5 I Wasser suspendiert werden. Die Suspension wird sprühgetrocknet. Hierzu wird der Sprühtrockner ( Mini Spray Dryer B-191 der Firma Büchi) wie folgt betrieben: Zerstäubungsluftstrom 650 Liter pro Stunde, Eingangstemperatur 140 °C, keine Düsenkühlung, Düsendurchmesser 0,7 mm, Aspirator 70 %, Pumpe 30 %. Es werden 197,5 g weißer Feststoff aus dem
Produkt auffang - Gefäß entnommen. Die Ausbeute beträgt 40 %. Ein Anteil von mehr als 90 % der Partikel liegen mit ihrem Durchmesser im Bereich von 1-10 µm.

Die Verwendbarkeit als Trägermaterial für eine Applikation als Dry Powder bei der inhalativen Gabe eines Wirkstoffs wird mittels der in Beispiel 7 beschriebenen Andersen Impactor-Apparatur bestimmt. Die Ergebnisse zeigen in Figur 8 die prinzipielle Lungengängigkeit der durch Sprühtrocknung erhaltenen Partikel (siehe auch Figuren 5 - 7 zu den Beispielen 7 und 8).

### Beispiel 17

### Charakterisierung der Fließfähigkeit von Mikropartikeln und Pulver aus Poly(1,4-alpha-D-glukan) nach List et al., Arzneiformenlehre, WVG, Stuttgart, 1985; Kap. 2.10.1

100 g Pulver aus Bsp. 3 und 4 sowie Bsp. 1 werden in einen Rieseltrichter (Rieselfähigkeits-Prüfgerät, Fa. Engelsmann, Ludwigshafen) gefüllt und dabei die Ausflußöffnung verschlossen gehalten. Die Ausflußöffnung wird freigegeben und das Pulver während des Auslaufens mit dem Rührbügel durch Betätigung einer Kurbel durchmischt. Nach 2 Minuten Ruhezeit werden der Durchmesser und die Höhe des Pulverkegels vermessen und daraus der Böschungswinkel bestimmt.
Bei zu hohem Schüttvolumen werden statt 100 g Pulver nur 50 g eingesetzt.

**Tabelle:**

| Es gilt: tan α = h / r nach List (supra, S. 53 ff) | | | |
|---|---|---|---|
| Trockenbindemittel | Kegelhöhe | Kegelradius | Böschungswinkel |
| Einheit | h in cm | r in cm | α in ° |
| Mikropartikel aus Bsp. 3, 4 | 6,4 | 8,50 | 37,0 |
| Pulver aus Bsp. 1 | 3,6 | 6,25 | 29,9 |
| Pulver aus Bsp. 1 | 4,1 | 6,75 | 31,3 |

Besonders geeignet sind Pulver mit einem Böschungswinkel ≤ 30°. Entsprechende referenzmessungen mit bekannt gut fkießenden Materialien wie EMDEX®, Avicel®, Toffena® ergeben Böschungswinkel um die 30° (Vgl. Bauer, Fröming, Führer, Phamazeutische Technologie, Stuttgart, 1989, S. 345).

### Beispiel 18:

### Beladung von Mikropartikeln hergestellt aus einer autoklavierten Poly (1-4-alpha-Dglukan)-Suspension

3,5g Poly(1-4-alpha-D-glukan)-Pulver werden dreimal mit 60°C heißem Wasser gewaschen. Anschließend wird in 200 ml entionisiertem Wasser suspendiert. Die Suspension wird in einem Laborautoklaven (Pressure Vessels; Typ 452 HCT 316; Fa. Parr Instruments Deutschland GmbH) angesetzt. Die Kammer wird mit Stickstoff gespült, so daß ein Druck von 1,5 bar erreicht wird.
Unter Rühren wird der Autoklav auf 130°C erwärmt. Die Autoklavierzeit bei dieser Temperatur beträgt 30 min. Es wird ein Druck von ca. 5 bar erzeugt.
Nach dem Abkühlen des Autoklavs wird die Suspension entnommen, 175 mg Theophyllin (entspricht 5% Beladung) darin gelöst und die wirkstoffhaltige Suspension sofort im Sprühtrockner versprüht. Dabei wird die Suspension mit Hilfe eines Magnetrührers kontinuierlich gerührt.
Der Sprühtrockner (Mini Sprühtrockner 191, Fa. Büchi, Schweiz) wird wie folgt betrieben: Zerstäubungsstickstoffstrom 700 Liter pro Stunde, Eingangstemperatur 220°C, eingeschaltete Düsenkühlung, Düsendurchmesser 0,5mm, Aspirator 70%, Pumpe 70%.
Das Sprühtrocknungsgut (Ausbeute mind. 25% bezogen auf die im Autoklaven eingesetzte Menge Feststoff) wird dem Auffanggefäß des Zyklons entnommen und trocken gelagert. Die Größe der Mikropartikel liegt zu über 90 % in einem Bereich kleiner 10 µm (Auswertung von Elektronenmikroskop-Aufnahmen).
Die Beladung der Mikropartikel mit Theophyllin wird photometrisch überprüft. Dazu werden 50 mg der Probe bei 60°C in 100 ml Dimethylsulfoxid gelöst und bei 271 nm vermessen (Lambda 20 Photometer,Perkin Elmer). Der Beladungsgrad liegt bei mind. 95%.
20 mg der wirkstoffbeladenen Mikropartikel werden in die Dosierkammer eines handelsüblichen Dry Powder Inhalers (Aerolizer®, Fa. Ciba-Geigy) gefüllt und die Flugweite wie in Beispiel 7 beschrieben mit dem Andersen-Impaktor vermessen.

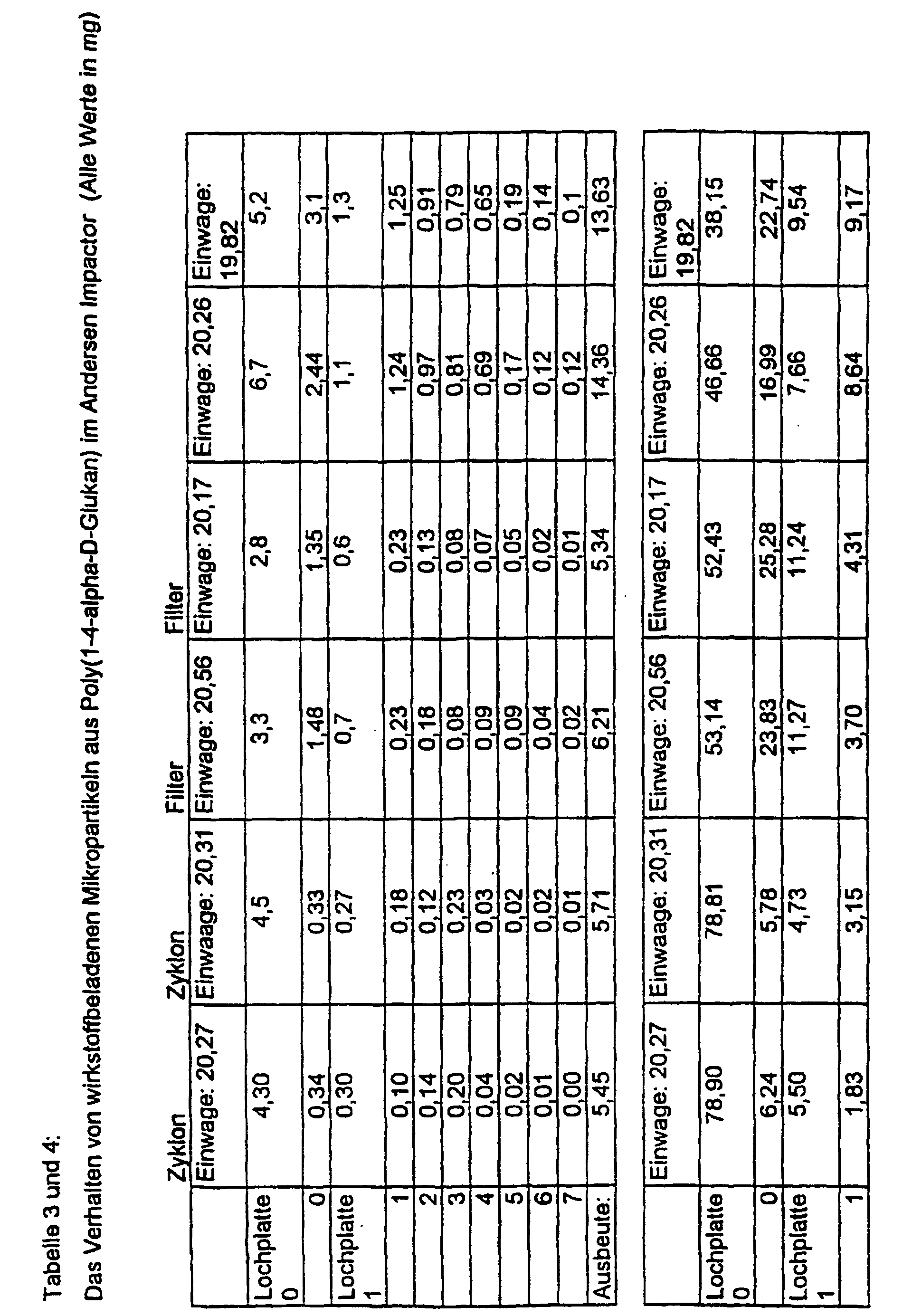

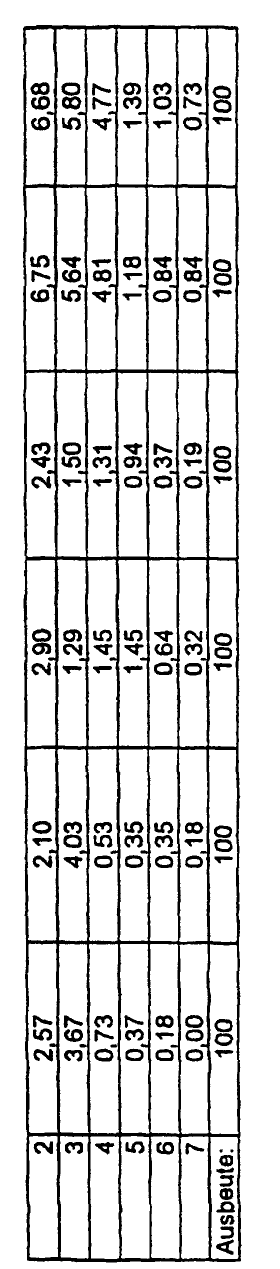

## Patentansprüche

1. Verwendung eines partikulären Wirkstoffträgers zur Herstellung von pharmazeutischen Zubereitungen zur pulmonalen Applikation, **dadurch gekennzeichnet, dass** der partikuläre Wirkstoffträger mindestens ein lineares wasserunlösliches Polysaccharid enthält und dessen mittlere Größe kleiner als 10µm ist.

2. Verwendung nach Anspruch 1, **dadurch gekennzeichnet, dass** der partikuläre Wirkstoffträger Agglomerate und/oder Cluster bildet, die kleiner als 60µm sind.

3. Verwendung nach Anspruch 1 oder 2, **dadurch gekennzeichnet, dass** der partikuläre Wirkstoffträger in einer Mischung vorliegt und eine effektive Wirkstoffverteilung und deren Penetration in der Lunge ermöglicht.

4. Verwendung nach einem der vorhergehenden Ansprüche, **dadurch gekennzeichnet, dass** der partikuläre Wirkstoffträger ganz oder teilweise aus mindestens einem wasserunlöslichen linearen Polysaccharid, welches in einem biotechnischen Verfahren hergestellt wurde, besteht.

5. Verwendung nach einem der vorhergehenden Ansprüche, **dadurch gekennzeichnet, dass** der partikuläre Wirkstoffträger ganz oder teilweise aus mindestens einem wasserunlöslichen linearen Polysaccharid, welches durch einen biokatalytischen Prozess hergestellt wurde, besteht.

6. Verwendung nach einem der vorhergehenden Ansprüche, **dadurch gekennzeichnet, dass** der partikuläre Wirkstoffträger ganz oder teilweise aus mindestens einem wasserunlöslichen linearen Polysaccharid, welches durch einen fermentativen Prozess hergestellt wurde, besteht.

7. Verwendung nach einem der Ansprüche 1 bis 6, **dadurch gekennzeichnet, dass** das lineare wasserunlösliche Polysaccharid Poly(1,4-α-D-glukan) ist.

8. Verwendung nach einem der Ansprüche 1 bis 7, **dadurch gekennzeichnet, dass** der partikuläre Wirkstoffträger biokompatibel und/oder pharmazeutisch akzeptabel ist.

9. Verwendung nach einem der Ansprüche 1 bis 8, **dadurch gekennzeichnet, dass** der partikuläre Wirkstoffträger seine Wirkstoffe kontrolliert in den Alveolen abgibt.

10. Verwendung nach einem der Ansprüche 1 bis 9, **dadurch gekennzeichnet, dass** der partikuläre Wirkstoffträger zusätzlich einen oder mehrere Wirkstoffe enthält.

11. Verwendung nach einem der Ansprüche 1 bis 10, **dadurch gekennzeichnet, dass** der partikuläre Wirkstoffträger mit Hilfe eines "Dry Powder Inhalers" inhalierbar ist.

12. Dispergierbare Trockenformulierung und/oder Puder enthaltend partikuläre Wirkstoffträger nach einem der Ansprüche 1 bis 10.

13. Dispergierbare Trockenformulierung und/oder Puder nach einem der Ansprüche 12 bis 22, **dadurch gekennzeichnet, dass** diese einen Wassergehalt von weniger als 25% aufweist, bevorzugt einen Wassergehalt von weniger als 15% und besonders bevorzugt einen Wassergehalt von 5-10%.

14. Dispergierbare Trockenformulierung und/oder Puder nach Anspruch 12 und 13 mit einem Böschungswinkel ≤ 30 Grad.

15. Pharmazeutische Zusammensetzung enthaltend lineare wasserunlösliche Polysaccharide nach einem der Ansprüche 1 bis 8 und 10 neben üblichen Trägerstoffen, Hilfsmitteln und/oder Zusatzstoffen.

## Claims

1. The use of a particulate excipient for the production of pharmaceutical preparations for pulmonary administration, wherein the particulate excipient comprises at least one linear water-insoluble polysaccharide and its average size is smaller than 10 µm.

2. Use as claimed in claim 1, wherein the particulate excipient forms agglomerates and/or clusters that are smaller than 60 µm.

3. Use as claimed in claim 1 or 2, wherein the particulate excipient is present in a mixture and enables effective active substance distribution and penetration thereof in the lungs.

4. Use as claimed in one of the preceding claims, wherein the particulate excipient consists entirely or partially of at least one water-insoluble linear polysaccharide that has been produced in a biotechnological process.

5. Use as claimed in one of the preceding claims, wherein the particulate excipient consists entirely or partially of at least one water-insoluble linear polysaccharide that has been produced by a biocatalytic process.

6. Use as claimed in one of the preceding claims, wherein the particulate excipient consists entirely or partially of at least one water-insoluble linear polysaccharide that has been produced by a fermentation process.

7. Use as claimed in one of claims 1 to 6, wherein the linear water-insoluble polysaccharide is poly(1,4-α-D-glucan).

8. Use as claimed in one of claims 1 to 7, wherein the particulate excipient is biocompatible and/or pharmaceutically acceptable.

9. Use as claimed in one of claims 1 to 8, wherein the particulate excipient provides a controlled release of its active substances in the alveoli.

10. Use as claimed in one of claims 1 to 9, wherein the particulate excipient additionally comprises one or more active substances.

11. Use as claimed in one of claims 1 to 10, wherein the particulate excipient is inhalable with the aid of a dry powder inhaler.

12. A dispersible dry formulation and/or powder comprising particulate excipients as claimed in one of claims 1 to 10.

13. A dispersible dry formulation and/or powder as claimed in one of claims 12 to 22, which has a water content of less than 25 %, preferably a water content of less than 15 % and particularly preferably a water content of 5 - 10 %.

14. A dispersible dry formulation and/or powder as claimed in claims 12 and 13 having an angle of repose ≤ 30 degrees.

15. A pharmaceutical composition comprising linear water-insoluble polysaccharides as claimed in one of claims 1 to 8 and 10 together with customary excipients, auxiliaries and/or additives.

## Revendications

1. Utilisation d'un véhicule particulaire de principes actifs pour la préparation de préparations pharmaceutiques pour applications pulmonaires, **caractérisée en ce que** le véhicule particulaire de principes actifs renferme au moins un polysaccharide linéaire insoluble dans l'eau et dont la taille moyenne de particules est inférieure à 10 µm.

2. Utilisation selon la revendication 1, **caractérisée en ce que** le véhicule particulaire de principes actifs forme des agglomérés et/ou amas, dont la taille est inférieure à 60 µm.

3. Utilisation selon la revendication 1 ou 2, **caractérisée en ce que** le véhicule particulaire de principes actifs est présent sous fcrme d'un mélange et permet d'obtenir une répartition efficace du principe actif et sa pénétration dans le poumon.

4. Utilisation selon l'une des revendications précédentes, **caractérisée en ce que** le véhicule particulaire de principes actifs est constitué entièrement ou partiellement par au moins un polysaccharide linéaire insoluble dans l'eau, que l'on prépare au moyen d'un procédé biotechnologique.

5. Utilisation selon l'une des revendications précédentes, **caractérisée en ce que** le véhicule particulaire de principes actifs est constitué entièrement ou partiellement par au moins un polysaccharide linéaire insoluble dans l'eau, que l'on prépare au moyen d'un procédé biocatalytique.

6. Utilisation selon l'une des revendications précédentes, **caractérisée en ce que** le véhicule particulaire de principes actifs est constitué entièrement ou partiellement par au moins un polysaccharide linéaire insoluble dans l'eau, que l'on prépare au moyen d'un procédé fermentatif.

7. Utilisation selon l'une des revendications 1 à 6, **caractérisée en ce que** le polysaccharide linéaire insoluble dans l'eau est le poly(1,4-α-D-glucane).

8. Utilisation selon l'une des revendications 1 à 7, **caractérisée en ce que** le véhicule particulaire de principes actifs est biocompatible et/ou acceptable en pharmacie.

9. Utilisation selon l'une des revendications 1 à 8, **caractérisée en ce que** le véhicule particulaire de principes actifs délivre ses principes actifs de manière contrôlée dans les alvéoles.

10. Utilisation selon l'une des revendications 1 à 9, **caractérisée en ce que** le véhicule particulaire de principes actifs contient de plus un ou plusieurs principes actifs.

11. Utilisation selon l'une des revendications 1 à 10, **caractérisée en ce que** le véhicule particulaire de principes actifs est inhalable à l'aide d'un "Dry Powder Inhalers".

12. Formulation sèche et/ou poudre dispersables renfermant des véhicules particulaires de principes actifs selon l'une des revendications 1 à 10.

13. Formulation sèche et/ou poudre dispersables selon l'une des revendications 12 à 22, **caractérisées en ce qu'**elles présentent une teneur en eau inférieure à 25 %, de préférence une teneur en eau inférieure à 15 % et de manière particulière une teneur en eau de 5 à 10 %.

14. Formulation sèche et/ou poudre dispersables selon la revendication 12 et 13 ayant un angle d'inclinaison ≤30 degrés.

15. Composition pharmaceutique renfermant des polysaccharides insolubles dans l'eau selon l'une des revendications 1 à 8 et 10, au côté des véhicules, adjuvants et/ou matières d'addition usuelles.
